(19) Europäisches Patentamt
European Patent Office
Office européen des brevets

(11) Numéro de publication : **0 017 523**
**B1**

# (12) FASCICULE DE BREVET EUROPÉEN

(45) Date de publication du fascicule du brevet : **01.09.82**

(21) Numéro de dépôt : **80400323.4**

(22) Date de dépôt : **12.03.80**
**Le dossier contient des informations techniques présentées postérieurement au dépôt de la demande et ne figurant pas dans le présent fascicule.**

(51) Int. Cl.³ : **C 07 D495/04**, C 07 D519/00, A 61 K 31/55// C07D295/18, C07D295/20, C07D339/00, (C07D495/04, 339/00, 209/00),(C07D519/00, 495/00, 471/00),(C07D495/04, 339/00, 307/00)

(54) **Nouveaux dérivés du dithiépinno (1,4)(2,3-c)pyrrole, leur préparation et les médicaments qui les contiennent.**

(30) Priorité : **15.03.79 FR 7906562**

(43) Date de publication de la demande : **15.10.80 (Bulletin 80/21)**

(45) Mention de la délivrance du brevet : **01.09.82 Bulletin 82/35**

(84) Etats contractants désignés : **AT BE CH DE FR GB IT LU NL SE**

(56) Documents cités : **DE - A - 2 707 072**

(73) Titulaire : **RHONE-POULENC INDUSTRIES**
**22 Avenue Montaigne**
**F-75008 Paris (FR)**

(72) Inventeur : **Farge, Daniel**
**30, rue des Pins Sylvestres**
**F-94320 Thiais (FR)**
Inventeur : **Leger, André**
**97, rue des Morillons**
**F-75015 Paris (FR)**
Inventeur : **Ponsinet, Gérard**
**16, rue Lachevardière**
**F-94370 Sucy-en-Brie (FR)**

(74) Mandataire : **Gaumont, Robert et al**
**RHONE-POULENC RECHERCHES Brevets PHARMA**
**25, Quai Paul Doumer**
**F-92408 Courbevoie Cedex (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 017 523 B1

Imprimerie Jouve, 18, rue St-Denis, 75001 Paris, France

Nouveaux dérivés du dithiépinno (1,4) (2,3-c)pyrrole, leur préparation et les médicaments qui les contiennent

La présente invention concerne de nouveaux dérivés du dithiépinno [1,4] [2,3-c] pyrrole de formule générale :

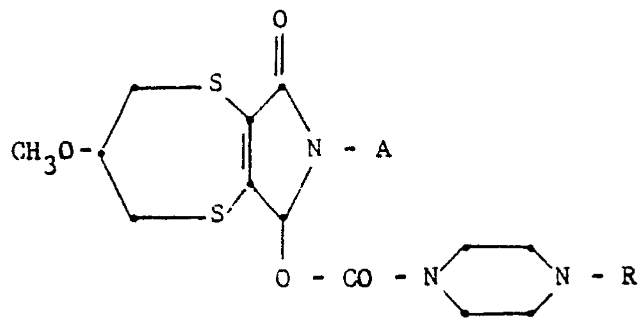

(I)

à l'état de bases libres ou de sels d'addition avec les acides, leur préparation et les médicaments qui les contiennent.

Dans la formule générale (I), A représente un radical pyridyl-2, quinolyl-2 ou naphtyridine-1,8 yl-2, ces radicaux étant éventuellement substitués par un atome d'halogène ou par un radical alcoyle contenant 1 à 4 atomes de carbone ou alcoyloxy contenant 1 à 4 atomes de carbone, et R représente un atome d'hydrogène ou un radical alcoyle contenant 1 à 4 atomes de carbone ou alcanoyle contenant 1 à 4 atomes de carbone.

Etant donné la présence de deux atomes de carbone asymétriques dans la molécule, les produits de formule générale (I), et leurs sels, peuvent exister sous la forme de diastéréoisomères ; la présente invention concerne les formes diastéréoisomères des produits de formule générale (I) ainsi que leurs mélanges.

Selon l'invention, les nouveaux produits de formule générale (I) peuvent être préparés par action d'une chlorocarbonylpipérazine de formule générale :

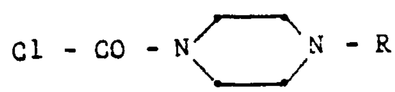

(II)

dans laquelle R est défini comme précédemment, sur un dérivé du dithiépinno [1,4] [2,3-c] pyrrole de formule générale :

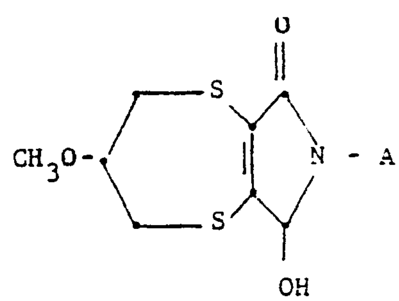

(III)

dans laquelle A est défini comme précédemment.

La réaction peut être effectuée en faisant réagir un produit de formule générale (II) sur un produit de formule générale (III) sous forme d'un sel alcalin, éventuellement préparé in situ, en opérant dans un solvant organique anhydre, tel que le diméthylformamide ou le tétrahydrofuranne, à une température inférieure à 60 °C.

On peut également faire réagir un produit de formule générale (II), éventuellement sous forme de sel (de préférence le chlorhydrate), sur un produit de formule générale (III) en opérant en présence de pyridine et éventuellement en présence d'une amine tertiaire telle que la triéthylamine qui libère le produit de formule générale (II) de son sel.

Le dérivé du dithiépinno [1,4] [2,3-c] pyrrole de formule générale (III) peut être obtenu par réduction partielle d'un imide de formule générale :

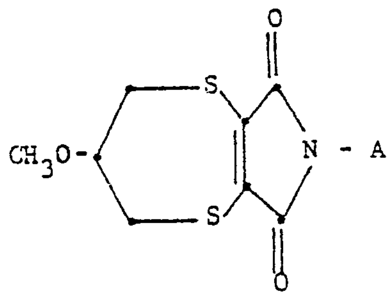 (IV)

dans laquelle A est défini comme précédemment.

Généralement la réduction s'effectue au moyen d'un borohydrure alcalin en opérant en solution organique ou hydroorganique, par exemple dans un mélange dioxanne-tétrahydrofuranne ou dioxanne-méthanol ou dioxanne-eau ou méthanol-eau ou éthanol-eau.

L'imide de formule générale (IV) peut être obtenu par action d'une amine de formule générale :

$$H_2N{-}A \qquad (V)$$

dans laquelle A est défini comme précédemment, sur l'anhydride de l'acide méthoxy-6 dihydro-6,7 5H-dithiépinne [1,4] dicarboxylique-2,3.

Généralement la réaction s'effectue par chauffage dans un solvant organique tel que l'acide acétique, le diméthylformamide, l'acétonitrile ou l'oxyde de phényle ou un mélange de ces solvants, en présence ou non d'un carbodiimide tel que le dicyclohexylcarbodiimide ou le (diéthylamino-3 propyl)-3 isopropyl-1 carbodiimide.

L'anhydride de l'acide méthoxy-6 dihydro-6,7 5H-dithiépinne [1,4] dicarboxylique-2,3 peut être préparé par action d'une base minérale telle que la soude puis d'un acide tel que l'acide chlorhydrique sur le méthoxy-3 dioxo-6,8 tétrahydro-3,4,7,8 2H, 6H-dithiépinno [1,4] [2,3-c] pyrrole.

Le métoxy-3 dioxo-6,8 tétrahydro-3,4,7,8 2H, 6H-dithiépinno [1,4] [2,3-c] pyrrole peut être obtenu par action de l'acide chlorhydrique anhydre sur une suspension de méthoxy-6 dihydro-6,7 5H-dithiépinne [1,4] dicarbonitrile-2,3 dans l'acide formique à une température inférieure à 28 °C.

Le méthoxy-6 dihydro-6,7 5H-dithiépinne [1,4] dicarbonitrile-2,3 peut être obtenu par action du dibromo-1,3 méthoxy-2 propane sur le sel disodique du dimercapto-2,3 maléonitrile en opérant dans un solvant organique tel que le diméthylformamide à une température voisine de 100 °C.

Le dibromo-1,3 méthoxy-2 propane peut être préparé selon le procédé décrit par D.E. Horning et coll., Can. J. Chem., *48*, 975-982 (1970).

Le sel disodique du dimercapto-2,3 maléonitrile peut être préparé selon le procédé décrit par H.R. Schweizer, Helv. Chim. Acta; *52*, 2228 (1969).

La pipérazine de formule générale (II) dans laquelle R représente un radical alcanoyle peut être obtenue par action du phosgène en solution toluénique, à une température voisine de – 5 °C, sur une pipérazine de formule générale :

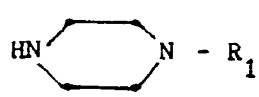 (VI)

dans laquelle $R_1$ représente le radical alcanoyle correspondant.

La pipérazine de formule générale (VI) peut être obtenue à partir de la pipérazine par application des méthodes habituelles de préparation des amides telles que l'action d'un acide de formule générale :

$$R_2{-}COOH \qquad (VII)$$

dans laquelle $R_2$ représente un atome d'hydrogène ou un radical alcoyle (contenant 1 à 3 atomes de carbone) ou d'un dérivé de cet article tel qu'un halogénure, un ester, l'anhydride, un anhydride mixte, l'amide ou l'azide, sur la pipérazine. Le produit de formule générale (VI) peut être séparé de la pipérazine disubstituée qui se forme simultanément, par application des méthodes physiques ou chimiques habituelles.

Selon l'invention, les nouveaux produits de formule générale (I) peuvent être préparés par action d'une pipérazine de formule générale :

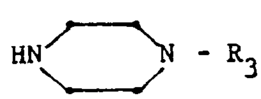 (VIII)

dans laquelle $R_3$ représente un atome d'hydrogène ou un radical alcoyle contenant 1 à 4 atomes de carbone ou d'une pipérazine de formule générale (VI) sur un carbonate mixte de formule générale :

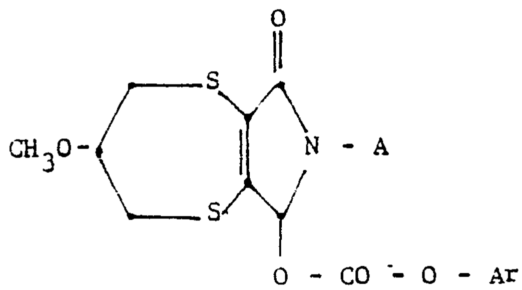

(IX)

dans laquelle A est défini comme précédemment et Ar représente un radical phényle éventuellement substitué par un radical alcoyle contenant 1 à 4 atomes de carbone ou par un radical nitro.

Genéralement la réaction s'effectue dans un solvant organique anhydre tel que l'acétonitrile à une température comprise entre 0 et 50 °C.

Le carbonate mixte de formule générale (IX) peut être obtenu par action d'un chloroformiate de formule générale :

$$Cl—CO—O—Ar \qquad (X)$$

dans laquelle Ar est défini comme précédemment, sur un produit de formule générale (III).

Généralement, la réaction s'effectue dans un solvant organique basique tel que la pyridine ou dans un solvant organique tel que le tétrahydrofuranne en présence d'un agent alcalin de condensation.

Selon l'invention, les nouveaux produits de formule générale (I) dans laquelle R représente un radical alcanoyle peuvent être préparés par action d'un acide de formule générale (VII) ou d'un dérivé réactif de cet acide tel qu'un halogénure (de préférence le chlorure), l'anhydride, un anhydride mixte, l'amide ou l'azide, sur un produit de formule générale (I) dans laquelle R représente un atome d'hydrogène, c'est-à-dire un produit de formule générale :

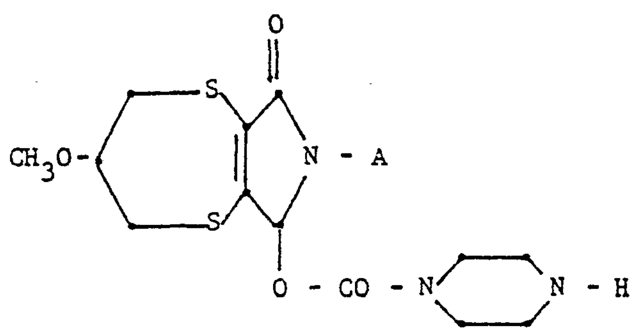

(XI)

dans laquelle A est défini comme précédemment.

Lorsque l'on utilise un acide de formule générale (VII), la réaction s'effectue généralement dans un solvant organique inerte tel que l'acétonitrile, le chlorure de méthylène, le diméthylformamide ou l'acétate d'éthyle en présence d'un agent de condensation tel que le dicyclohexylcarbodiimide ou le N,N-carbonyldiimidazole à une température comprise entre 20 et 60 °C.

Lorsque l'on utilise un halogénure de l'acide de formule générale (VII), de préférence le chlorure, la réaction s'effectue dans un solvant organique tel que le chlorure de méthylène en présence d'un accepteur d'acide tel que la pyridine ou la triméthylamine à une température comprise entre 0 et 30 °C.

Lorsque l'on utilise l'anhydride de l'acide de formule générale (VII) ou un anhydride mixte, la réaction s'effectue généralement par chauffage à une température comprise entre 30 et 100 °C.

Lorsque l'on utilise l'amide de l'acide de formule générale (VII), la réaction s'effectue généralement par chauffage à une température supérieure à 100 °C, éventuellement dans un solvant organique tel qu'un hydrocarbure aromatique et de préférence en présence d'iode.

Lorsque l'on utilise l'amide de l'acide de formule générale (VII), la réaction s'effectue généralement dans un solvant organique tel que le dioxanne en présence d'oxyde de magnésium à une température comprise entre 25 et 60 °C.

Le produit de formule générale (XI) peut être obtenu par action de la chlorocarbonylpipérazine sur un

produit de formule générale (III) ou par action de la pipérazine sur un carbonate mixte de formule générale (IX).

Le produit de formule générale (XI) peut aussi être obtenu à partir d'un produit de formule générale :

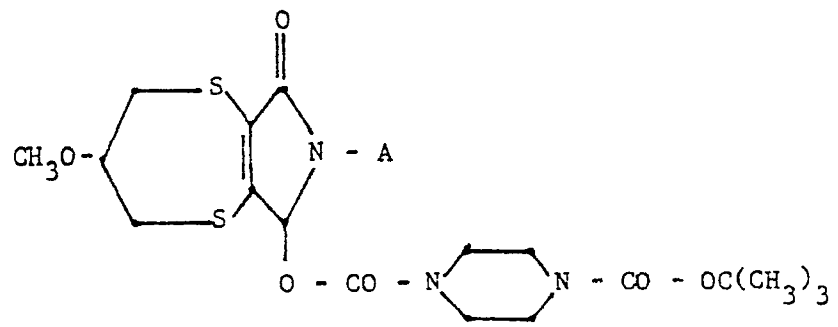

(XII)

dans laquelle A est défini comme précédemment, par traitement au moyen d'acide trifluoroacétique à une température comprise entre 0 et − 10 °C.

Le produit de formule générale (XII) peut être obtenu par action de la chlorocarbonyl-4 tertiobutyloxycarbonyl-1 pipérazine sur un produit de formule générale (III).

La réaction s'effectue généralement dans un solvant organique tel que le chlorure de méthylène en présence d'un accepteur d'acide tel que la pyridine ou la triéthylamine à une température comprise entre 0 et 30 °C.

La chlorocarbonyl-4 tertiobutyloxycarbonyl-1 pipérazine peut être obtenue par action du phosgène en solution toluénique, à une température voisine de − 5°C, sur la tertiobutyloxycarbonyl-1 pipérazine.

La tertiobutyloxycarbonyl-1 pipérazine peut être obtenue par action du chlorhydrate de pipérazine sur l'azidoformiate de tertiobutyle.

Les formes diastéréoisomères des produits de formule générale (I) peuvent être séparées de leurs mélanges par application de méthodes physico-chimiques telles que la cristallisation fractionnée.

Les nouveaux produits de formule générale (I) peuvent être éventuellement purifiés par des méthodes physiques (telles que la cristallisation ou la chromatographie) ou chimiques (telles que la formation d'un sel, cristallisation de celui-ci puis décomposition en milieu alcalin ; dans ces opérations, la nature de l'anion du sel est indifférente, la seule condition étant que le sel soit bien défini et aisément cristallisable).

Les nouveaux produits de formule générale (I), et plus particulièrement ceux pour lesquels R représente un atome d'hydrogène ou un radical alcoyle peuvent être transformés en sels d'addition avec les acides.

Les sels d'addition peuvent être obtenus par action des nouveaux composés sur des acides dans des solvants appropriés.

Comme solvants organiques on utilise par exemple des alcools, des cétones, des éthers ou des solvants chlorés. Le sel formé précipité, après concentration éventuelle de sa solution, et il est séparé par filtration ou décantation.

On connaît déjà par le brevet français 2 341 312 des dérivés du dithiépinno [1,4] [2,3-c] pyrrole actifs comme tranquillisants, anticonvulsivants, décontracturants et hypnogènes.

Il a été trouvé que les produits selon l'invention, dont la structure diffère de celle des produits de l'art antérieur par la présence d'un groupement méthoxy sur le cycle dithiépinno-pyrrole, présentent une activité supérieure à celle de leurs homologues non méthoxylés.

Les nouveaux produits selon l'invention et éventuellement leurs sels d'addition avec les acides présentent donc des propriétés pharmacologiques intéressantes. Ils se sont montrés particulièrement actifs comme tranquillisants, anticonvulsivants, décontracturants et hypnogènes.

Chez l'animal (souris), ils se sont montrés actifs à des doses comprises entre 0,5 et 25 mg/kg p.o. en particulier dans les tests suivants :

— bataille électrique selon une technique voisine de celle de Tedeshi et coll., J. Pharmacol., *125*, 28 (1959)

— convulsion au pentétrazol selon une technique voisine de celle de Everett et Richards, J. Pharmacol., *81*, 402 (1944)

— électrochoc supramaximal selon la technique de Swinyard et coll., J. Pharmacol. *106*, 319 (1952)

— mortalité à la strychnine selon une technique voisine de celle de F. Barzaghi et coll., Arzneimittel-Forschung, *23*, 683 (1973), et

— activité locomotrice selon la technique de Courvoisier, Congrès des Médecins Aliénistes et Neurologistes - Tours - 8-13 juin 1959 et Julou, Bulletin de la Société de Pharmacie de Lille, n° 2, janvier 1967, p. 7.

Par ailleurs, ces produits présentent l'avantage de manifester une longue durée d'action.

Les produits selon l'invention présentent une faible toxicité : leur $DL_{50}$ p.o. (souris) est généralement supérieure à 900 mg/kg.

D'un intérêt tout particulier sont les produits de formule générale (I) dans laquelle A représente un radical pyridyl-2 ou naphtyridine-1,8 yl-2 éventuellement substitué par un atome d'halogène ou par un radical alcoxy dont la partie alcoyle contient 1 à 4 atomes de carbone et R représente un radical alcoyle contenant 1 à 4 atomes de carbone, notamment le (chloro-7 naphtyridine-1,8 yl-2)-7 méthoxy-3 (méthyl-4 pipérazinyl-1) carbonyloxy-6 oxo-8 tétrahydro-3,4,7,8 2H, 6H-dithiépinno [1,4] [2,3-c] pyrrole.

Pour l'emploi médicinal, il est fait usage des nouveaux composés soit à l'écart de base, soit à l'écart de sels d'addition avec les acides pharmaceutiquement acceptables, c'est-à-dire non toxiques aux doses d'utilisation.

Comme exemples de sels d'addition pharmaceutiquement acceptables peuvent être cités des sels d'acides minéraux (tels que les chlorhydrates, sulfates, nitrates, phosphates) ou organiques (tels que les acétates, propionates, succinates, benzoates, fumarates, maléates, théophylline-acétates, salicylates, phénolphtalinates, méthylène-bis-ß-oxynaphtoates) ou des dérivés de substitution de ces composés.

Les exemples suivants, donnés à titre non limitatif, montrent comment l'invention peut être mise en pratique.

Exemple 1

A une suspension de 7,4 g de (chloro-5 pyridyl-2)-7 hydroxy-6 méthoxy-3 oxo-8 tétrahydro-3,4,7,8 2H, 6H-dithiépinno [1,4] [2,3-c] pyrrole et de 43,0 g de chlorhydrate de chlorocarbonyl-1 méthyl-4 pipérazine dans 215 $cm^3$ de chlorure de méthylène anhydre on ajoute, vers 15 °C, 42 $cm^3$ de triéthylamine puis 90 $cm^3$ de pyridine anhydre. Le mélange réactionnel est chauffé pendant 10 heures à 45 °C. Après refroidissement le mélange est dilué par addition de 250 $cm^3$ de chlorure de méthylène. La phase organique est lavée 3 fois par 300 $cm^3$ au total d'une solution aqueuse de soude 1 N, 3 fois par 750 $cm^3$ au total d'eau distillée, séchée sur du sulfate de sodium anhydre et évaporée. Le résidu est dissous dans 50 $cm^3$ d'éthanol bouillant. Après 20 heures de refroidissement à 2 °C les cristaux apparus sont séparés par filtration, lavés 3 fois par 30 $cm^3$ au total d'éthanol glacé et séchés sous pression réduite (20 mm de mercure). Le produit obtenu (5,1 g ; F. = 197 °C) est dissous dans 80 $cm^3$ d'éthanol bouillant. Après filtration de la solution bouillante puis 16 heures de refroidissement à 2 °C, les cristaux apparus sont séparés par filtration, lavés par 8 $cm^3$ d'éthanol glacé et séchés sous pression réduite (0,2 mm de mercure) à 55 °C. On obtient 3,2 g de (chloro-5 pyridyl-2)-7 méthoxy-3 (méthyl-4 pipérazinyl-1) carbonyloxy-6 oxo-8 tétrahydro-3,4,7,8 2H, 6H-dithiépinno [1,4] [2,3-c] pyrrole fondant à 199 °C.

Le (chloro-5 pyridyl-2)-7 hydroxy-6 méthoxy-3 oxo-8 tétrahydro-3,4,7,8 2H, 6H-dithiépinno [1,4] [2,3-c] pyrrole de départ peut être préparé de la façon suivante :

— Préparation du sel disodique du dimercapto-2,3 maléonitrile selon H.R. Schweizer, Helv. Chim. Acta *52*, 2228 (1969).

— Préparation du dibromo-1,3 méthoxy-2 propane ($Eb_{28}$ = 98 °C) selon D.E. Horning et coll., Can. J. Chem. *48*, 975-982 (1970).

— Préparation de 44,4 g de méthoxy-6 dihydro-6,7 5H-dithiépinne [1,4]-dicarbonitrile-2,3 (F. = 105 °C) par action de 116 g de dibromo-1,3 méthoxy-2 propane sur 155 g de sel disodique du dimercapto-2,3 maléonitrile dans 1,25 litre de diméthylformamide vers 95-100 °C.

— Préparation de 237 g de méthoxy-3 dioxo-6,8 tétrahydro-3,4,7,8 2H, 6H-dithiépinno [1,4] [2,3-c] pyrrole (F. = 185 °C) par action d'acide chlorhydrique anhydre sur une suspension de 217 g de méthoxy-6 dihydro-6,7 5H-dithiépinne [1,4]-dicarbonitrile-2,3 dans 2 600 $cm^3$ d'acide formique à 28 °C maximum.

— Préparation de 162,3 g d'anhydride de l'acide méthoxy-6 dihydro-6,7 5H-dithiépinne [1,4]-dicarboxylique-2,3 (F. = 141 °C), par action de 222 $cm^3$ de solution aqueuse de soude 5,4 N dans 1 100 $cm^3$ d'eau distillée, au reflux, pendant 30 minutes, puis de 150 $cm^3$ d'une solution aqueuse d'acide chlorhydrique 11,8 N, sur 237 g de méthoxy-3 dioxo-6,8 tétrahydro-3,4,7,8 2H, 6H-dithiépinno [1,4] [2,3-c] pyrrole.

— Préparation de 13,5 g de (chloro-5 pyridyl-2)-7 méthoxy-3 dioxo-6,8 tétrahydro-3,4,7,8 2H, 6H-dithiépinno [1,4] [2,3-c] pyrrole (F. = 217 °C) par action de 6,4 g d'amino-2 chloro-5 pyridine sur 11,6 g d'anhydride de l'acide méthoxy-6 dihydro-6,7 6H-dithiépinne [1,4] dicarboxylique 2,3 dans 25 $cm^3$ d'oxyde de diphényle, à 170-180 °C, pendant 1 heure en présence de 0,4 $cm^3$ d'acide acétique.

— Préparation de 7,5 g de (chloro-5 pyridyl-2)-7 hydroxy-6 méthoxy-3 oxo-8 tétrahydro-3,4,7,8 2H, 6H-dithiépinno [1,4] [2,3-c] pyrrole (F. = 147 °C) par action de 1,8 g de borohydrure de potassium sur 13,5 g de (chloro-5 pyridyl-2)-7 méthoxy-3 dioxo-6,8 tétrahydro-3,4,7,8 2H, 6H-dithiépinno [1,4] [2,3-c] pyrrole dans un mélange de 120 $cm^3$ de tétrahydrofuranne et 36 $cm^3$ de méthanol entre − 10 °C et + 20 °C.

Exemple 2

A une suspension de 6,0 g de (chloro-7 quinolyl-2)-7 hydroxy-6 méthoxy-3 oxo-8 tétrahydro-3,4,7,8 2H, 6H-dithiépinno [1,4] [2,3-c] pyrrole et de 30,2 g de chlorhydrate de chlorocarbonyl-1 méthyl-4 pipérazine dans 150 $cm^3$ de chlorure de méthylène anhydre on ajoute, vers 15 °C, 29,5 $cm^3$ de

triéthylamine puis 65 $cm^3$ de pyridine anhydre. Le mélange réactionnel est chauffé pendant 10 heures à 45 °C. Après refroidissement le mélange est dilué par addition de 300 $cm^3$ de chlorure de méthylène. La phase organique est lavée 2 fois par 600 $cm^3$ au total d'une solution aqueuse de soude 1 N, 5 fois par 1 000 $cm^3$ au total d'eau distillée, séchée sur du sulfate de sodium et évaporée. Le résidu est traité par 50 $cm^3$ d'éthanol bouillant. Après 1 heure de refroidissement à 2 °C, les cristaux sont séparés par filtration, lavés 2 fois par 20 $cm^3$ au total d'éthanol glacé et séchés sous pression réduite (20 mm de mercure). Le produit obtenu (4,6 g ; F. = 228 °C) est dissous dans un mélange bouillant de 50 $cm^3$ d'acétonitrile et 150 $cm^3$ d'éthanol. Après 16 heures de refroidissement à 2 °C, les cristaux apparus sont séparés par filtration, lavés par 5 $cm^3$ d'éthanol glacé et séchés sous pression réduite (0,2 mm de mercure) à 55 °C. On obtient 3,3 g de (chloro-7 quinolyl-2)-7 méthoxy-3 (méthyl-4 pipérazinyl-1) carbonyloxy-6 oxo-8 tétrahydro-3,4,7,8 2H, 6H-dithiépinno [1,4] [2,3-c] pyrrole fondant à 28 °C.

Le (chloro-7 quinolyl-2)-7 hydroxy-6 méthoxy-3 oxo-8 tétrahydro-3,4,7,8 2H, 6H-dithiépinno [1,4] [2,3-c] pyrrole de départ peut être préparé de la façon suivante :

— Préparation de 6,1 g de (chloro-7 quinolyl-2)-7 méthoxy-3 dioxo-6,8 tétrahydro-3,4,7,8 2H, 6H-dithiépinno [1,4] [2,3-c] pyrrole (F. = 170 °C) par action de 3,6 g d'amino-2 chloro-7 quinoléine sur 4,65 g d'anhydride de l'acide méthoxy-6 dihydro-6,7 5H-dithiépinne [1,4] dicarboxylique-2,3 dans 10 $cm^3$ d'oxyde de diphényle, à 170-180 °C, pendant 1/2 heure, en présence de 0,1 $cm^3$ d'acide acétique.

— Préparation de 6,0 g de (chloro-7 quinolyl-2)-7 hydroxy-6 méthoxy-3 oxo-8 tétrahydro-3,4,7,8 2H, 6H-dithiépinno [1,4] [2,3-c] pyrrole par action de 0,46 g de borohydrure de sodium sur 6,0 g de (chloro-7 quinolyl-2)-7 méthoxy-3 dioxo-6,8 tétrahydro-3,4,7,8 2H, 6H-dithiépinno [1,4] [2,3-c] pyrrole dans un mélange de 52 $cm^3$ de tétrahydrofuranne et 20 $cm^3$ de méthanol entre − 12 °C et + 2 °C.

Exemple 3

A une suspension de 36,0 g de (chloro-7 naphtyridine-1,8 yl-2)-7 hydroxy-6 méthoxy-3 oxo-8 tétrahydro-3,4,7,8 2H, 6H-dithiépinno [1,4] [2,3-c] pyrrole et de 162 g de chlorhydrate de chlorocarbonyl-1 méthyl-4 pipérazine dans 900 $cm^3$ de chlorure de méthylène anhydre on ajoute, vers 15 °C, 144 $cm^3$ de triéthylamine puis 360 $cm^3$ de pyridine anhydre. Le mélange réactionnel est chauffé pendant 5 heures à 45 °C. Après refroidissement le mélange est dilué par addition de 900 $cm^3$ de chlorure de méthylène. La phase organique est lavée 3 fois par 750 $cm^3$ au total d'eau distillée, séchée sur du sulfate de sodium anhydre et évaporée. Le résidu est traité par 750 $cm^3$ d'éthanol bouillant. Après 2 heures de refroidissement à 2 °C, les cristaux sont séparés par filtration, lavés par 100 $cm^3$ d'éthanol glacé puis deux fois par 200 $cm^3$ au total d'eau distillée et séchés à l'air. Le produit obtenu (36,5 g ; F. = 260 °C) est dissous dans 3 000 $cm^3$ d'acétonitrile bouillant. Après 3 heures de refroidissement à 20 °C les cristaux apparus sont séparés par filtration, lavés deux fois par 200 $cm^3$ au total d'acétonitrile et séchés sous pression réduite (0,2 mm de mercure) à 50 °C. On obtient 26,6 g de (chloro-7 naphtyridine-1,8 yl-2)-7 méthoxy-3 (méthyl-4 pipérazinyl-1) carbonyloxy-6 oxo-8 tétrahydro-3,4,7,8 2H, 6H-dithiépinno [1,4] [2,3-c] pyrrole fondant à 250 °C (sous forme d'un mélange en quantités pratiquement égales des formes A et B des diastéréoisomères).

Le (chloro-7 naphtyridine-1,8 yl-2)-7 hydroxy-6 méthoxy-3 oxo-8 tétrahydro-3,4,7,8 2H, 6H-dithiépinno [1,4] [2,3-c] pyrrole de départ peut être préparé de la façon suivante :

— Préparation de 72,6 g de (chloro-7 naphtyridine-1,8 yl-2)-7 méthoxy-3 dioxo-6,8 tétrahydro-3,4,7,8 2H, 6H-dithiépinno [1,4] [2,3-c] pyrrole (F. = 264 °C) par action de 48,3 g d'amino-2 chloro-7 naphtyridine-1,8 et de 53,0 g de (diéthylamino-3 propyl)-3 isopropyl-1 carbodiimide sur 62,4 g d'anhydride de l'acide méthoxy-6 dihydro-6,7 5H-dithiépinne [1,4] dicarboxylique-2,3 dans 625 $cm^3$ d'acétonitrile, pendant 8 heures, à 55-60 °C.

— Préparation de 130,8 g de (chloro-7 naphtyridine-1,8 yl-2)-7 hydroxy-6 méthoxy-3 oxo-8 tétrahydro-3,4,7,8 2H, 6H-dithiépinno [1,4] [2,3-c] pyrrole (F. = 150 °C) par action d'une solution de 19,7 g d'hydroborure de potassium dans 450 $cm^3$ d'eau distillée sur 143 g de (chloro-7 naphtyridine-1,8 yl-2)-7 méthoxy-3 dioxo-6,8 tétrahydro-3,4,7,8 2H, 6H-dithiépinno [1,4] [2,3-c] pyrrole dans 1 800 $cm^3$ deméthanol, entre 30 et 35 °C pendant 1 heure 1/2.

Le mélange des formes A et B des diastéréoisomères peut être séparé de la façon suivante :

— Préparation de 36,6 g de mélange 1 : 1 selon le procédé décrit ci-dessus (la proportion des diastéréoisomères est déterminée par analyse chromatographique sur couche mince à haute performance).

Obtention de la forme A

On effectue des cristallisations fractionnées en opérant selon le tableau suivant :

(Voir Tableau Page 8)

| N° de recristallisation | Solvant utilisé | Durée de recristallisation | Solvant de lavage | Poids obtenu | Composition % | |
|---|---|---|---|---|---|---|
| | | | | | forme A | forme B |
| 1 | Acétonitrile : 1360 cm3<br>DMF : 340 cm3 | 3 heures à 20°C | Acétonitrile : 40 cm3 | 23,0 g | 70 | 30 |
| 2 | Acétonitrile : 2400 cm3 | 1 heure à 20°C<br>3 heures à 2°C | Acétonitrile : 40 cm3 | 15,4 g | 80 | 20 |
| 3 | Acétonitrile : 1620 cm3<br>DMF : 180 cm3 | 1 heure à 20°C<br>3 heures à 2°C | Acétonitrile : 60 cm3 | 10,6 g | 87 | 13 |
| 4 | Acétonitrile : 1080 cm3<br>DMF : 120 cm3 | 1 heure à 20°C<br>3 heures à 2°C | Acétonitrile : 60 cm3 | 8,6 g | 92 | 8 |
| 5 | Acétonitrile : 900 cm3<br>DMF : 100 cm3 | 1 heure à 20°C<br>3 heures à 2°C | Acétonitrile : 50 cm3 | 6,8 g | 96 | 4 |
| 6 | Acétonitrile : 828 cm3<br>DMF : 92 cm3 | 1 heure à 20°C<br>3 heures à 2°C | Acétonitrile : 50 cm3 | 5,6 g | >97 | <3 |

(DMF = diméthylformamide)

Après séchage à 80 °C sous pression réduite (0,2 mm de mercure) on obtient 5,6 g de (chloro-7 naphtyridine-1,8 yl-2)-7 méthoxy-3 (méthyl-4 pipérazinyl-1) carbonyloxy-6 oxo-8 tétrahydro-3,4,7,8 2H, 6H-dithiépinno [1,4] [2,3-c] pyrrole, forme A, contenant moins de 3 % de la forme B, fondant à 288 °C.

Les eaux-mères de la recristallisation n° 1 sont évaporées à sec sous pression réduite (20 mm, puis 0,2 mm de mercure) à une température inférieure à 50 °C. Les cristaux (13,6 g) sont recristallisés 6 fois dans l'acétonitrile comme indiqué dans le tableau d'obtention de la forme B, les durées de recristallisation sont identiques pour chacune des recristallisations (1 heure à 20 °C puis 3 heures à 2 °C).

Les eaux-mères de la recristallisation n° 2 de l'obtention de la forme A sont évaporées à sec sous pression réduite (20 mm, puis 0,2 mm de mercure) à une température inférieure à 50 °C. Les cristaux obtenus (7,0 g) sont recyclés à la recristallisation n° 5 de l'obtention de la forme B.

Obtention de la forme B

| N° de recristallisation | Quantité d'acétonitrile | Acétonitrile de lavage | Poids obtenu | Composition % | |
|---|---|---|---|---|---|
| | | | | forme A | forme B |
| 1 | 1300 cm3 | 30 cm3 | 11,0 g | 30 | 70 |
| 2 | 900 cm3 | 20 cm3 | 8,1 g | <30 | >70 |
| 3 | 650 cm3 | 20 cm3 | 6,2 g | 25 | 75 |
| 4 | 550 cm3 | 20 cm3 | 4,8 g | 20 | 80 |
| 5 | 1300 cm3 | 40 cm3 | 5,55 g | < 10 | >90 |
| 6 | 600 cm3 | 20 cm3 | 4,2 g | < 2 | >98 |

Après séchage à 60 °C sous pression réduite (0,2 mm de mercure), on obtient 4,2 g de (chloro-7 naphtyridine-1,8 yl-2)-7 méthoxy-3 (méthyl-4 pipérazinyl-1) carbonyloxy-6 oxo-8 tétrahydro-3,4,7,8 2H, 6H-dithiépinno [1,4] [2,3-c] pyrrole, forme B, contenant moins de 2 % de la forme A, fondant à 255 °C.

Exemple 4

On opère comme à l'exemple 3 mais à partir de 7,9 g de (chloro-7 naphtyridine-1,8 yl-2)-7 hydroxy-6 méthoxy-3 oxo-8 tétrahydro-3,4,7,8 2H, 6H-dithiépinno [1,4] [2,3-c] pyrrole et de 8,2 g de chlorocarbonyl-1 propionyl-4 pipérazine dans un mélange de 100 cm$^3$ de chlorure de méthylène et de 40 cm$^3$ de pyridine anhydre en présence de 2,8 cm$^3$ de triéthylamine, à 45 °C pendant 2 heures. On obtient, après recristallisation dans 140 cm$^3$ d'acétonitrile, 6,5 g de (chloro-7 naphtyridine-1,8 yl-2)-7 méthoxy-3 oxo-8 (propionyl-4 pipérazinyl-1) carbonyloxy-6 tétrahydro-3,4,7,8 2H, 6H-dithiépinno [1,4] [2,3-c] pyrrole fondant à 216 °C.

La chlorocarbonyl-1 propionyl-4 pipérazine peut être préparée de la façon suivante :

— Préparation de 1 020 g de propionyl-1 pipérazine ($Eb_1$ = 110 °C ; chlorhydrate : F. = 167 °C) par action de 1 135 g de pipérazine anhydre sur 965 g de propionamide dans 965 cm$^3$ de xylène anhydre, au reflux, pendant 48 heures, en présence de 8 g d'iode bisublimé.

— Préparation de 552 g de chloroformyl-1 propionyl-4 pipérazine (huile) par action de 297 g de phosgène sur 852 g de propionyl-1 pipérazine dans 12 litres de toluène anhydre à 0 °C.

Exemple 5

A une suspension de 10,7 g d'hydroxy-6 méthoxy-3 (méthoxy-7 naphtyridine-1,8 yl-2)-7 oxo-8 tétrahydro-3,4,7,8 2H, 6H-dithiépinno [1,4] [2,3-c] pyrrole et de 16,4 g de chlorhydrate de chlorocarbonyl-1 méthyl-4 pipérazine dans 440 cm$^3$ de chlorure de méthylène anhydre on ajoute, vers 15 °C, 34,5 cm$^3$ de triéthylamine puis 195 cm$^3$ de pyridine anhydre. Le mélange réactionnel est chauffé pendant 3 heures à 45 °C. Après refroidissement le mélange est dilué par addition de 300 cm$^3$ de chlorure de méthylène. La phase organique est lavée 3 fois par 600 cm$^3$ au total d'eau distillée, séchée sur du sulfate de sodium anhydre et évaporée. Le résidu obtenu (41,0 g) est traité par 150 cm$^3$ de méthanol bouillant. Après 1 heure de refroidissement à 2 °C, les cristaux sont séparés par filtration, lavés 2 fois par 40 cm$^3$ au total de méthanol glacé et séchés sous pression réduite (20 mm de mercure) à 20 °C. Le produit obtenu (9,0 g ;

F. = 215 °C) est dissous dans 175 $cm^3$ de méthanol bouillant. Après 2 heures de refroidissement à 2 °C les cristaux apparus sont séparés par filtration, lavés 2 fois par 20 $cm^3$ au total de méthanol glacé et séchés sous pression réduite (0,2 mm de mercure) à 50 °C. On obtient 7,4 g de méthoxy-3 (méthoxy-7 naphtyridine-1,8 yl-2)-7 (méthyl-4 pipérazinyl-1) carbonyloxy-6 oxo-8 tétrahydro-3,4,7,8 2H, 6H-dithiépinno [1,4] [2,3-c] pyrrole fondant à 218 °C.

L'hydroxy-6 méthoxy-3 (méthoxy-7 naphtyridine-1,8 yl-2)-7 oxo-8 tétrahydro-3,4,7,8 2H, 6H-dithiépinno [1,4] [2,3-c] pyrrole de départ peut être préparé de la façon suivante :

— Préparation de 11,6 g de dioxo-6,8 méthoxy-3 (méthoxy-7 naphtyridine-1,8 yl-2)-7 tétrahydro-3,4,7,8 2H, 6H-dithiépinno [1,4] [2,3-c] pyrrole (F. = 196 °C) par action de 7,5 g d'amino-2 méthoxy-7 naphtyridine-1,8 et de 8,5 g de (diéthylamino-3 propyl)-3 isopropyl-1 carbodiimide sur 10,0 g d'anhydride de l'acide méthoxy-6 dihydro-6,7 5H-dithiépinne [1,4] dicarboxylique-2,3 dans 150 $cm^3$ d'acétonitrile, au reflux, pendant 4 heures 1/2.

— Préparation de 10,7 g d'hydroxy-6 méthoxy-3 (méthoxy-7 naphtyridine-1,8 yl-2)-7 oxo-8 tétrahydro-3,4,7,8 2H, 6H-dithiépinno [1,4] [2,3-c] pyrrole (F. = 192 °C) par action de 1,32 g de borohydrure de potassium sur 12,7 g de dioxo-6,8 méthoxy-3 (méthoxy-7 naphtyridine-1,8 yl-2)-7 tétrahydro-3,4,7,8 2H, 6H-dithiépinno [1,4] [2,3-c] pyrrole dans le mélange de 100 $cm^3$ de tétrahydrofuranne et de 30 $cm^3$ de méthanol entre − 10 °C et 0 °C.

Exemple 6

A une suspension de 7,3 g d'hydroxy-6 méthoxy-3 (méthyl-7 naphtyridine-1,8 yl-2)-7 oxo-8 tétrahydro-3,4,7,8 2H, 6H-dithiépinno [1,4] [2,3-c] pyrrole et de 11,7 g de chlorhydrate de chlorocarbonyl-1 méthyl-4 pipérazine dans 300 $cm^3$ de chlorure de méthylène anhydre on ajoute, vers 15 °C, 24,5 $cm^3$ de triéthylamine puis 130 $cm^3$ de pyridine anhydre. On chauffe à 45 °C pendant 3 heures puis on ajoute 6,0 g de chlorhydrate de chlorocarbonyl-1 méthyl-4 pipérazine et on chauffe encore pendant 1/2 heure à 45 °C. Après refroidissement le mélange est dilué par addition de 300 $cm^3$ de chlorure de méthylène. La phase organique est lavée 3 fois par 600 $cm^3$ au total d'eau distillée, séchée sur du sulfate de sodium et évaporée. Le résidu obtenu (25,0 g) est dissous dans 70 $cm^3$ d'acétonitrile bouillant. Après 48 heures de refroidissement à 2 °C les cristaux apparus sont séparés par filtration, lavés 2 fois par 20 $cm^3$ au total d'acétonitrile glacé et séchés sous pression réduite (20 mm de mercure) à 20 °C. Le produit obtenu (5,25 g ; F. = 220 °C) est dissous dans 100 $cm^3$ de chlorure de méthylène, la solution est filtrée sur 200 g de silice (0,2-0,5 mm) contenus dans une colonne de 2,1 cm de diamètre. On élue avec 400 $m^3$ de chlorure de méthylène ; cet éluat est éliminé. L'élution est poursuivie avec un mélange de 784 $cm^3$ de chlorure de méthylène et 16 $cm^3$ de méthanol : cet éluat est évaporé sous pression réduite (20 mm de mercure) à une température inférieure à 60 °C. Le résidu est séché sous pression réduite (0,2 mm de mercure) à 50 °C. On obtient 4,2 g de méthoxy-3 (méthyl-7 naphtyridine-1,8 yl-2)-7 (méthyl-4 pipérazinyl-1) carbonyloxy-6 oxo-8 tétrahydro-3,4,7,8 2H, 6H-dithiépinno [1,4] [2,3-c] pyrrole fondant à 225 °C.

L'hydroxy-6 méthoxy-3 (méthyl-7 naphtyridine-1,8 yl-2)-7 oxo-8 tétrahydro-3,4,7,8 2H, 6H-dithiépinno [1,4] [2,3-c] pyrrole de départ peut être préparé de la façon suivante :

— Préparation de 7,7 g de dioxo-6,8 méthoxy-3 (méthyl-7 naphtyridine-1,8 yl-2)-7 tétrahydro-3,4,7,8 2H, 6H-dithiépinno [1,4] [2,3-c] pyrrole (F. = 245 °C) par action de 5,9 g d'amino-2 méthyl-7 naphtyridine-1,8 et de 8,1 g de (diéthylamino-3 propyl)-3 isopropyl-1 carbodiimide sur 8,6 g d'anhydride de l'acide méthoxy-6 dihydro-6,7 5H-dithiépinne [1,4] dicarboxylique-2,3 dans 130 $cm^3$ d'acétonitrile au reflux pendant 6 heures.

— Préparation de 7,3 g d'hydroxy-6 méthoxy-3 (méthyl-7 naphtyridine-1,8 yl-2)-7 oxo-8 tétrahydro-3,4,7,8 2H, 6H-dithiépinno [1,4] [2,3-c] pyrrole (F. = 170 °C) par action de 1,13 g de borohydrure de potassium sur 10,4 g de dioxo-6,8 méthoxy-3 (méthyl-7 naphtyridine-1,8 yl-2)-7 tétrahydro-3,4,7,8 2H, 6H-dithiépinno [1,4] [2,3-c] pyrrole dans le mélange de 80 $cm^3$ de tétrahydrofuranne et de 24 $cm^3$ de méthanol entre − 10 °C et 0 °C.

Exemple 7

A une suspension de 11,15 g de carbonate de [(chloro-5 pyridyl-2)-7 méthoxy-3 oxo-8 tétrahydro-3,4,7,8 2H, 6H-dithiépinno [1,4] [2,3-c] pyrrolyle-6] et de phényle dans 80 $cm^3$ d'acétonitrile anhydre on ajoute, à 20 °C, une solution de 6,8 g de propionyl-1 pipérazine dans 50 $cm^3$ d'acétonitrile anhydre. Le mélange réactionnel est chauffé au reflux pendant 6 heures. L'acétonitrile est évaporé sous pression réduite (20 mm de mercure ; 2,67 kPa). Le résidu est traité par 100 $cm^3$ d'eau et extrait 3 fois par 210 $cm^3$ au total de chlorure de méthylène ; on lave les extraits organiques 2 fois par 200 $cm^3$ au total d'eau distillée, sèche sur du sulfate de sodium anhydre et évapore à sec. Le résidu obtenu (14,7 g ; huileux) est dissous dans 50 $cm^3$ d'un mélange de chloroforme et de méthanol (97 - 3 en volume). La solution est chromatographiée sur 400 g de silice (0,04-0,063 mm), contenue dans une colonne de 6 cm de diamètre, sous une pression de 140 kPa. On élue avec le mélange de solvants défini précédemment sous la pression de 140 kPa. On recueille 1 800 $cm^3$ d'éluat qui est éliminé, puis 600 $cm^3$ d'éluat qu'on évapore à sec sous pression réduite (20 mm de mercure ; 2,67 kPa). Le résidu partiellement cristallisé obtenu (11,0 g) est dissous dans un mélange bouillant de 10 $cm^3$ d'acétonitrile et 50 $cm^3$ d'oxyde d'isopropyle. Après 2 heures de refroidissement à 2 °C, les cristaux apparus sont séparés par filtration, lavés 2 fois par

20 cm$^3$ au total d'oxyde d'isopropyle et séchés sous pression réduite (20 mm de mercure ; 2,67 kPa) à 20 °C. Le produit obtenu (4,1 g ; F. = 160 °C) est dissous dans 60 cm$^3$ d'acétonitrile bouillant. Après 2 heures de refroidissement à 2 °C, les cristaux apparus sont séparés par filtration, lavés 2 fois par 80 cm$^3$ au total d'oxyde d'isopropyle et séchés sous pression réduite (0,2 mm de mercure : 0,026 kPa) à 50 °C. On obtient 1,2 g de (chloro-5 pyridyl-2)-7 méthoxy-3 oxo-8 (propionyl-4 pipérazinyl-1) carbonyloxy-6 tétrahydro-3,4,7,8 2H, 6H-dithiépinno [1,4] [2,3-c] pyrrole fondant à 226 °C.

Le carbonate de [(chloro-5 pyridyl-2)-7 méthoxy-3 oxo-8 tétrahydro-3,4,7,8 2H, 6H-dithiépinno [1,4] [2,3-c] pyrrolyle-6] et de phénynyle peut être préparé de la façon suivante :

Par action de 16,7 g de chloroformiate de phényle sur 12,3 g de (chloro-5 pyridyl-2)-7 hydroxy-6 méthoxy-3 oxo-8 tétrahydro-3,4,7,8 2H, 6H-dithiépinno [1,4] [2,3-c] pyrrole (préparé comme décrit à l'exemple 1) dans 100 cm$^3$ de pyridine anhydre entre − 10 °C et + 20 °C, on obtient 10,1 g de carbonate de [(chloro-5 pyridyl-2)-7 méthoxy-3 oxo-8 tétrahydro-3,4,7,8 2H, 6H-dithiépinno [1,4] [2,3-c] pyrrolyle-6] et de phényle (F. = 167 °C).

La chlorocarbonyl-1 propionyl-4 pipérazine peut être préparée comme à l'exemple 5.

Exemple 8

A une suspension de 8,0 g de (chloro-7 naphtyridine-1,8 yl-2)-7 méthoxy-3 oxo-8 (pipérazinyl-1) carbonyloxy-6 tétrahydro-3,4,7,8 2H, 6H-dithiépinno [1,4] [2,3-c] pyrrole et de 4,85 g de dicyclohexylcarbodiimide dans 160 cm$^3$ de chlorure de méthylène anhydre on ajoute en 5 minutes, à 20 °C, 2,20 cm$^3$ d'acide isobutyrique. Le mélange réactionnel est agité pendant 1 heure à 20 °C. On sépare par filtration la dicyclohexylurée formée et lave 2 fois par 20 cm$^3$ au total de chlorure de méthylène. Le filtrat est évaporé à sec sous pression réduite (20 mm de mercure ; 2,67 kPa). Les cristaux obtenus sont traités par 80 cm$^3$ d'éthanol bouillant : on filtre à chaud, lave les cristaux insolubles par 10 cm$^3$ d'éthanol bouillant et sèche à l'air. Le produit obtenu (7,6 g ; F. = 205 °C) est dissous dans 80 cm$^3$ d'acétonitrile bouillant ; on filtre la solution bouillante. Après 4 heures de refroidissement à 2 °C, les cristaux apparus sont séparés par filtration, lavés 2 fois par 15 cm$^3$ au total d'acétonitrile glacé et séchés sous pression réduite (0,2 mm de mercure ; 0,026 kPa) à 60 °C. On obtient ainsi 4,6 g de (chloro-7 naphtyridine-1,8 yl-2)-7 (isobutyryl-4 pipérazinyl-1) carbonyloxy-6 méthoxy-3 oxo-8 tétrahydro-3,4,7,8 2H, 6H-dithiépinno [1,4] [2,3-c] pyrrole fondant à 232 °C.

Le (chloro-7 naphtyridine-1,8 yl-2)-7 méthoxy-3 oxo-8 (pipérazinyl-1) carbonyloxy-6 tétrahydro-3,4,7,8 2H, 6H-dithiépinno [1,4] [2,3-c] pyrrole de départ peut être séparé de la façon suivante :

— Préparation de 32,0 g de (t. butyloxycarbonyl-4 pipérazinyl-1) carbonyloxy-6 (chloro-7 naphtyridine-1,8 yl-2)-7 méthoxy-3 oxo-8 tétrahydro-3,4,7,8 2H, 6H-dithiépinno [1,4] [2,3-c] pyrrole (F. = 210 °C) par action de 27,4 g de (chlorocarbonyl-4 pipérazinyl-1) carboxylate de t. butyle sur 27,2 g de (chloro-7 naphtyridine-1,8 yl-2)-7 hydroxy-6 méthoxy-3 oxo-8 tétrahydro-3,4,7,8 2h, 6h-dithiépinno [1,4] [2,3-c] pyrrole (préparé comme décrit à l'exemple 3) dans 280 cm$^3$ de chlorure de méthylène, en présence de 140 cm$^3$ de pyridine anhydre et de 9,7 cm$^3$ de triéthylamine, vers 50 °C, pendant 8 heures.

— Préparation de 13,0 g de (chloro-7 naphtyridine-1,8 yl-2)-7 méthoxy-3 oxo-8 (pipérazinyl-1) carbonyloxy-6 tétrahydro-3,4,7,8 2H, 6H-dithiépinno [1,4] [2,3-c] pyrrole (F. vers 320 °C) par action de 190 cm$^3$ d'acide trifluoroacétique sur 32,0 g de (t. butyloxycarbonyl-4 pipérazinyl-1) carbonyloxy-6 (chloro-7 naphtyridine-1,8 yl-2)-7 méthoxy-3 oxo-8 tétrahydro-3,4,7,8 2H, 6H-dithiépinno [1,4] [2,3-c] pyrrole pendant 1 heure à − 5 °C.

Le (chlorocarbonyl-4 pipérazinyl-1) carboxylate de t. butyle peut être préparé de la manière suivante :

— Préparation de 91,0 g de pipérazinyl-1 carboxylate de t. butyle (F. = 60 °C) par action de 259,0 g de t. butyloxycarbonylazide sur 310,0 g de monochlorhydrate de pipérazine dans un mélange eau-dioxanne (1-2 en volumes) à 45 °C.

— Préparation de 24,8 g de (chlorocarbonyl-4 pipérazinyl-1) carboxylate de t. butyle (F. = 99 °C) par action de 11,0 g de phosgène sur 40,8 g de pipérazinyl-1 carboxylate de t. butyle dans le toluène à − 5 °C.

Exemple 9

En opérant comme à l'exemple 8 mais à partir de 1,0 g de (chloro-7 naphtyridine-1,8 yl-2)-7 méthoxy-3 oxo-8 (pipérazinyl-1) carbonyloxy-6 tétrahydro-3,4,7,8 2H, 6H-dithiépinno [1,4] [2,3-c] pyrrole, de 0,61 g de dicyclohexylcarbodiimide et de 0,22 cm$^3$ d'acide propionique dans 20 cm$^3$ de chlorure de méthylène, on obtient 0,57 g de (chloro-7 naphtyridine-1,8 yl-2)-7 méthoxy-3 oxo-8 (propionyl-4 pipérazinyl-1) carbonyloxy-6 tétrahydro-3,4,7,8 2H, 6H-dithiépinno [1,4] [2,3-c] pyrrole fondant à 216 °C.

La présente invention concerne également les médicaments qui contiennent les produits de formule générale (I) à l'état pur ou sous forme de compositions dans lesquelles ils sont associés à un adjuvant, un diluant et/ou un enrobage compatibles et pharmaceutiquement acceptables. Ces médicaments peuvent être employés par voie orale, rectale, parentérale ou percutanée.

Comme compositions solides pour administration orale peuvent être utilisés des comprimés, des pillules, des poudres (généralement dans des capsules de gélatine) ou des granulés. Dans ces compositions, le produit actif selon l'invention est mélangé à un ou plusieurs diluants inertes, tels que

saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions selon l'invention pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive ou des esters organiques injectables, par exemple l'oléate d'éthyle. Ces compositions peuvent également contenir des adjuvants, en particulier des agents mouillants, émulsifiants et dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont des suppositoires qui peuvent contenir, outre le produit actif, des excipients tels que le beurre de cacao ou la suppo-cire.

Les compositions pour administration percutanée sont les crèmes, pommades, lotions et liniments, dans lesquels le produit actif est associé à des excipients liquides ou pâteux, de préférence en association avec un véhicule favorisant la migration percutanée.

Les médicaments et compositions selon l'invention sont particulièrement utiles en thérapeutique humaine par leur action tranquillisante, anticovulsivante, décontractante et hypnogène.

En thérapeutique humaine, les doses dépendent de l'effet recherché et de la durée du traitement ; elles sont généralement comprises entre 0,5 et 25 mg par jour par voie orale pour un adulte.

D'une façon générale, le médecin déterminera la posologie qu'il estime la plus appropirée en fonction de l'âge, du poids et de tous les autres facteurs propres au sujet à traiter.

L'exemple suivant, donné à titre non limitatif, illustre une composition selon l'invention.

Exemple

On prépare selon la technique habituelle des comprimés dosés à 5 mg de produit actif ayant la composition suivante :

| | |
|---|---|
| — (chloro-7 naphtyridine-1,8 yl-2)-7 méthoxy-3 (méthyl-4 pipérazinyl-1) carbonyloxy-6 oxo-8 tétrahydro-3,4,7,8 2H, 8H-dithiépinno [1,4] [2,3-c] pyrrole, forme A. | 0,005 g |
| — amidon | 0,100 g |
| — silice précipité | 0,018 g |
| — stéarate de magnésium | 0,002 g |

**Revendications** (pour les Etats contractants : BE, CH, DE, FR, GB, IT, LU, NL, SE)

1. Un nouveau dérivé du dithiépinno [1,4] [2,3-c] pyrrole caractérisé en ce qu'il répond à la formule générale :

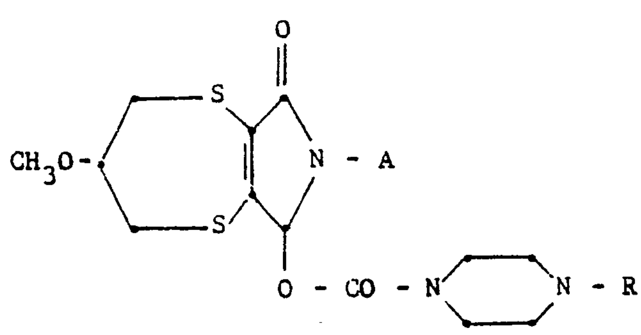

dans laquelle A représente un radical pyridyl-2, quinolyl-2 ou naphtyridine-1,8 yl-2, ces radicaux étant éventuellement substitués par un atome d'halogène ou par un radical alcoyle contenant 1 à 4 atomes de carbone ou alcoyloxy dont la partie alcoyle contient 1 à 4 atomes de carbone, et R représente un atome d'hydrogène ou un radical alcoyle contenant 1 à 4 atomes de carbone ou alcanoyle contenant 1 à 4 atomes de carbone, ses formes diastéréoisomères et leurs mélanges, à l'état de base libre ou de sels d'addition avec les acides.

2. Un procédé de préparation d'un produit selon la revendication 1 caractérisé en ce que l'on fait réagir une chlorocarbonylpipérazine de formule générale :

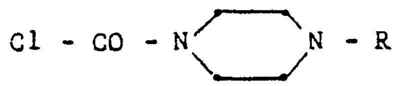

dans laquelle R est défini comme dans la revendication 1, sur un dérivé du dithiépinno [1,4] [2,3-c] pyrrole de formule générale :

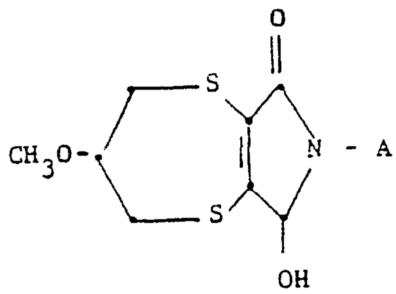

dans laquelle A est défini comme dans la revendication 1 puis éventuellement sépare le produit obtenu en ses formes diastéréoisomères et éventuellement le transforme en un sel d'addition avec un acide.

3. Un procédé selon la revendication 2 caractérisé en ce que l'on fait réagir une chlorocarbonylpipérazine définie comme dans la revendication 2 sur un dérivé du dithiépinno [1,4] [2,3-c] pyrrole sous forme de sel alcalin éventuellement préparé in situ, en opérant dans un solvant organique, puis éventuellement sépare le produit obtenu en ses formes diastéréoisomères et éventuellement le transforme en un sel d'addition avec un acide.

4. Un procédé selon la revendication 2 caractérisé en ce que l'on fait réagir une chlorocarbonylpipérazine définie comme dans la revendication 2, éventuellement sous forme de sel, sur un dérivé du dithiépinno [1,4] [2,3-c] pyrrole défini comme dans la revendication 2 en opérant en présence de pyridine et éventuellement en présence d'une base organique tertiaire, puis éventuellement sépare le produit obtenu en ses formes diastéréoisomères et éventuellement le transforme en un sel d'addition avec un acide.

5. Un procédé de préparation d'un produit selon la revendication 1 caractérisé en ce que l'on fait réagir une pipérazine de formule générale :

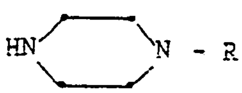

dans laquelle R est défini comme dans la revendication 1, sur un carbonate mixte de formule générale :

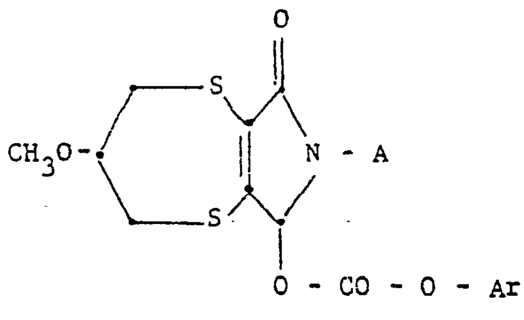

dans laquelle A est défini comme dans la revendication 1 et Ar représente un radical phényle éventuellement substitué par un radical alcoyle contenant 1 à 4 atomes de carbone ou par un radical nitro, puis éventuellement sépare le produit obtenu en ses formes diastéréoisomères et éventuellement le transforme en un sel d'addition avec un acide.

6. Un procédé de préparation d'un produit selon la revendication 1 pour lequel R représente un radical alcanoyle contenant 1 à 4 atomes de carbone, caractérisé en ce que l'on fait réagir un acide ou un

dérivé réactif d'un acide de formule générale :

$R_2$—COOH

dans laquelle $R_2$ représente un atome d'hydrogène ou un radical alcoyle contenant 1 à 3 atomes de carbone sur un produit de formule générale :

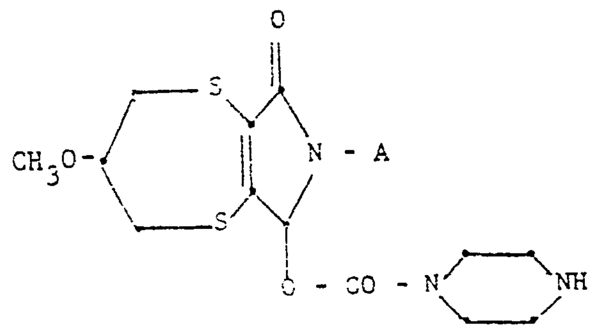

dans laquelle A est défini comme dans la revendication 1 puis éventuellement sépare le produit obtenu en ses formes diastéréoisomères et éventuellement le transforme en un sel d'addition avec un acide.

7. Un procédé de préparation d'un produit selon la revendication 1 pour lequel R représente un atome d'hydrogène, caractérisé en ce que l'on soumet à l'action de l'acide trifluoroacétique un produit de formule générale :

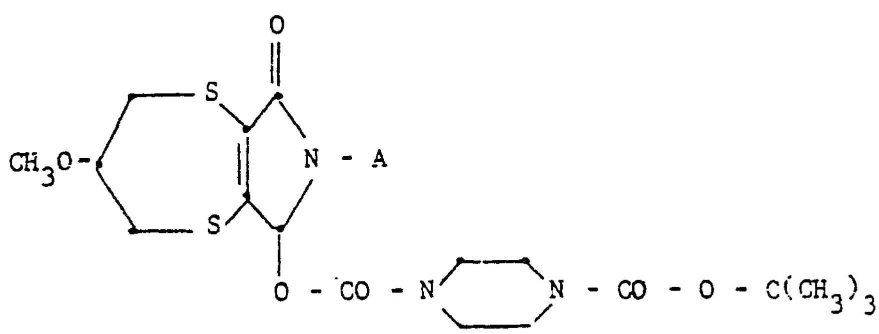

dans laquelle A est défini comme dans la revendication 1 puis éventuellement sépare le produit obtenu en ses formes diastéréoisomères et éventuellement le transforme en un sel d'addition avec un acide.

8. Médicament caractérisé en ce qu'il contient un produit selon la revendication 1 à l'état pur ou en association avec un ou plusieurs diluants ou adjuvants compatibles et pharmaceutiquement acceptables.

**Revendication** (pour l'Etat contractant AT)

Procédé de préparation d'un nouveau dérivé du dithiépinno [1,4] [2,3-c] pyrrole de formule générale :

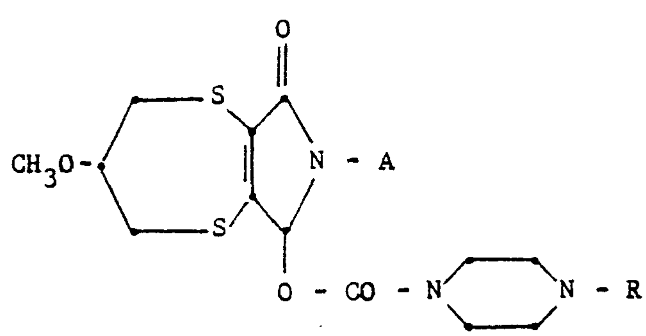

dans laquelle A représente un radical pyridyl-2, quinolyl-2 ou naphtyridine-1,8 yl-2, ces radicaux étant éventuellement substitués par un atome d'halogène ou par un radical alcoyle contenant 1 à 4 atomes de carbone ou alcoyloxy dont la partie alcoyle contient 1 à 4 atomes de carbone, et R représente un atome d'hydrogène ou un radical alcoyle contenant 1 à 4 atomes de carbone ou alcanoyle contenant 1 à 4 atomes de carbone, ses formes diastéréoisomères et leurs mélanges, à l'état de base libre ou de sels

d'addition avec les acides, caractérisé en ce que l'on fait réagir une chlorocarbonylpipérazine de formule générale :

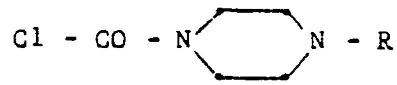

dans laquelle R est défini comme précédemment, sur un dérivé du dithiépinno [1,4] [2,3-c] pyrrole de formule générale :

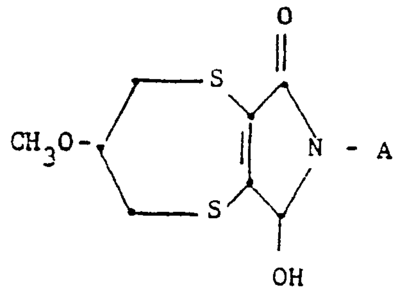

dans laquelle A est défini comme précédemment, ou en ce que l'on fait réagir une pipérazine de formule générale :

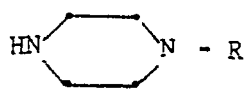

dans laquelle R est défini comme précédemment, sur un carbonate mixte de formule générale :

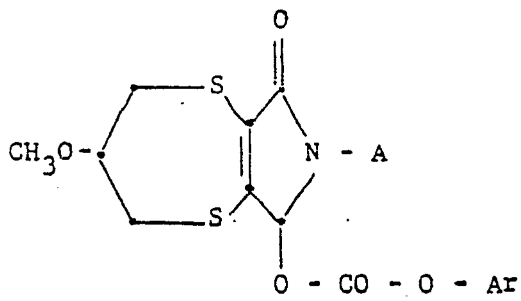

dans laquelle A est défini comme précédemment et Ar représente un radical phényle éventuellement substitué par un radical alcoyle contenant 1 à 4 atomes de carbone ou par un radical nitro ou, lorsque R représente un radical alcanoyle contenant 1 à 4 atomes de carbone, on fait réagir un acide ou un dérivé réactif d'un acide de formule générale :

$R_2$—COOH

dans laquelle $R_2$ représente un atome d'hydrogène ou un radical alcoyle contenant 1 à 3 atomes de carbone sur un produit de formule générale :

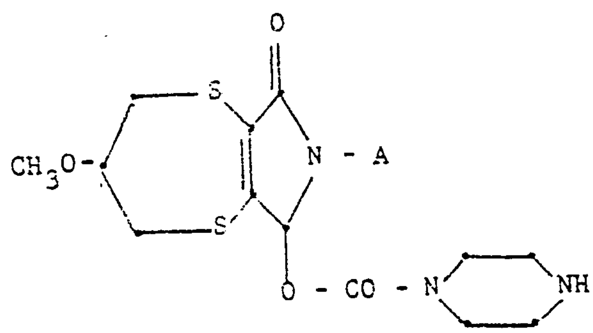

dans laquelle A est défini comme précédemment ou, lorsque R représente un atome d'hydrogène, on

soumet à l'action de l'acide trifluoroacétique un produit de formule générale :

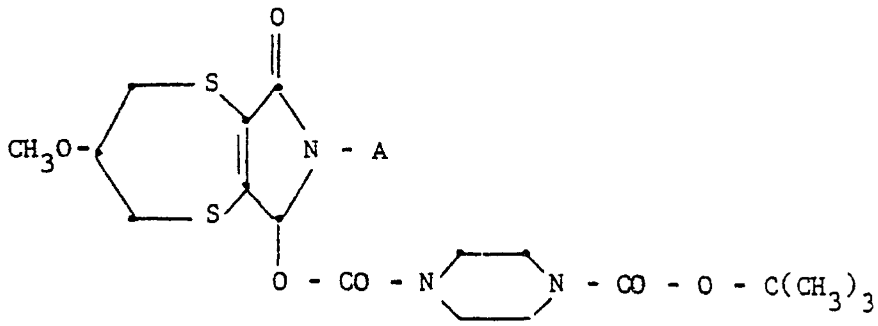

dans laquelle A est défini comme précédemment, puis éventuellement sépare le produit obtenu en ses formes diastéréoisomères et éventuellement le transforme en un sel d'addition avec un acide.

**Claims** (for the contracting States : BE, CH, DE, FR, GB, IT, LU, NL, SE)

1. A novel derivative of dithiepino [1,4] [2,3-c] pyrole, characterised in that it corresponds to the general formula :

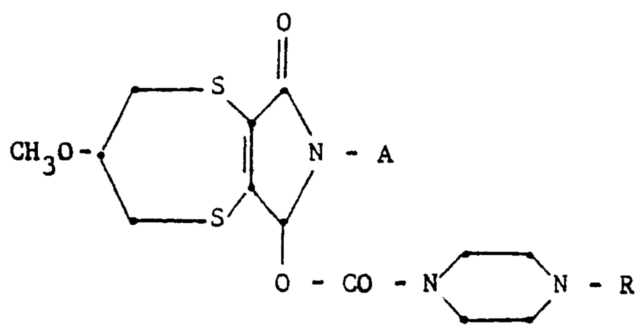

in which A represents a pyrid-2-yl, quinol-2-yl or 1,8-naphthyridin-2-yl radical, these radicals being optionally substituted by a halogen atom or an alkyl radical containing 1 to 4 carbon atoms or by an alkoxy radical of which the alkyl part contains 1 to 4 carbon atoms, and R represents a hydrogen atom or alkyl radical containing 1 to 4 carbon atoms, or an alkanoyl radical containing 1 to 4 carbon atoms, its diastereoisomeric forms and their mixtures, in the form of a free base or of addition salts with acids.

2. A process for the preparation of a product according to Claim 1, characterised in that a chlorocarbonylpiperazine of the general formula :

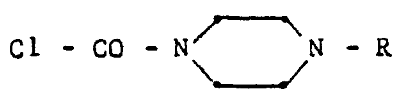

in which R is defined as in Claim 1, is reated with a dithiepino [1,4] [2,3-c] pyrrole derivative of the general formula :

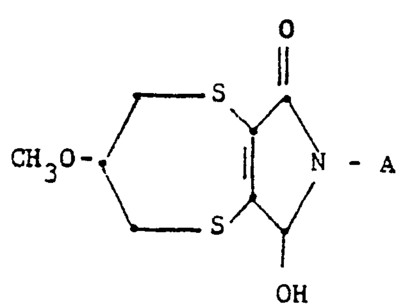

in which A is defined as in Claim 1 and thereafter, if desired, the product obtained is separated into its diastereoisomeric forms and, if desired, is converted to an addition salt wird an acid.

3. A process according to Claim 2, characterised in that a chlorocarbonylpiperazine defined as in Claim 2 is reacted with a dithiepino [1,4] [2,3-c] pyrrole derivative in the form of an alkali metal salt

(prepared in situ if desired), the reaction being carried out in an organic solvent, and thereafter, if desired, the product obtained is separated into its diastereoisomeric forms and, if desired, is converted to an addition salt with an acid.

4. A process according to Claim 2, characterised in that a chlorocarbonylpiperazine defined as in Claim 2, if appropriate in the form of a salt, is reacted with a dithiepino [1,4] [2,3-c] pyrrole derivative defined as in Claim 2 in the presence of pyridine and, if appropriate, in the presence of a tertiary organic base, and thereafter, if desired, the product obtained is separated into its diastereoisomeric forms and, if desired, is converted to an addition salt with an acid.

5. A process for the preparation of a product according to Claim 1, characterised in that a piperazine of the general formula :

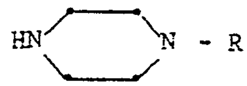

in which R is defined as in Claim 1 is reacted with a mixed carbonate of the general formula :

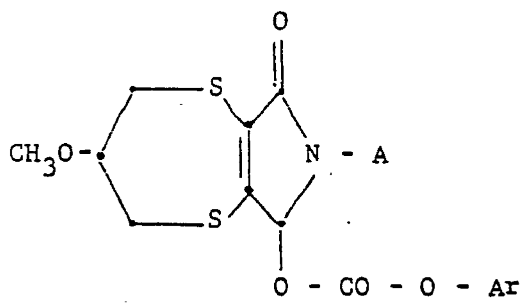

in which A is defined as in Claim 1 and Ar represents a phenyl radical which is optionally substituted by an alkyl radical containing 1 to 4 carbon atoms or by a nitro radical, and thereafter, if desired, the product obtained is separated into its diastereoisomeric forms and, if desired, is converted to an addition salt with an acid.

6. A process for the preparation of a product according to Claim 1, in which R represents an alkanoyl radical containing 1 to 4 carbon atoms, characterised in that an acid or a reactive derivative of an acid of the general formula :

$$R_2\text{—}COOH$$

in which $R_2$ represents a hydrogen atom or an alkyl radical containing 1 to 3 carbon atoms is reacted with a product of the general formula :

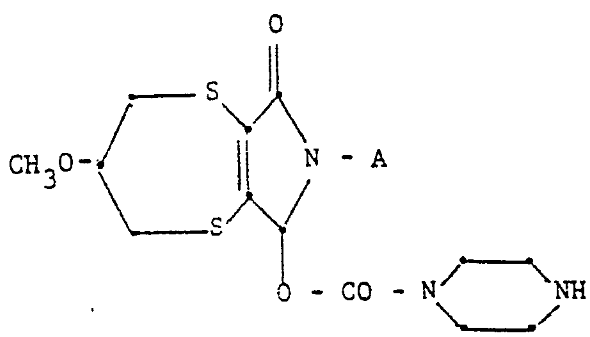

in which A is defined as in Claim 1, and thereafter, if desired, the product obtained is separated into its diastereoisomeric forms and, if desired, is converted to an addition salt with an acid.

7. A process for the preparation of a product according to Claim 1, in which R represents a hydrogen atom, characterised in that a product of the general formula :

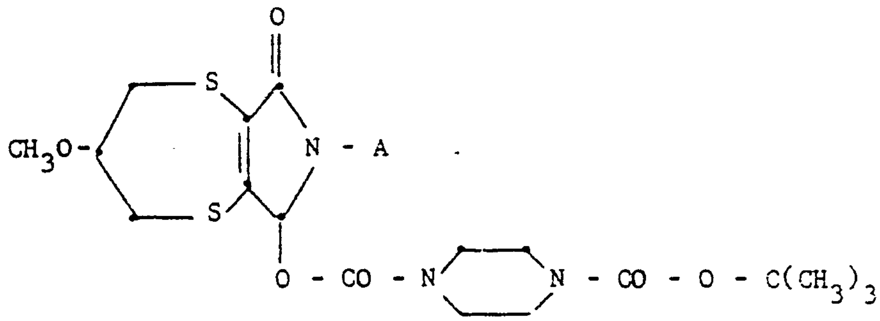

in which A is defined as in Claim 1 is subjected to the action of trifluoroacetic acid and thereafter, if desired, the product obtained is separated into its diastereoisomeric forms and, if desired, is converted to an addition salt with an acid.

8. A medicament, characterised in that it contains a product according to Claim 1 in the pure state or together with one or more compatible and pharmaceutically acceptable diluents or adjuvants.

**Claim** (for the contracting State AT)

A process for the preparation of a novel derivative of dithiepino [1,4] [2,3-c] pyrrole of general formula :

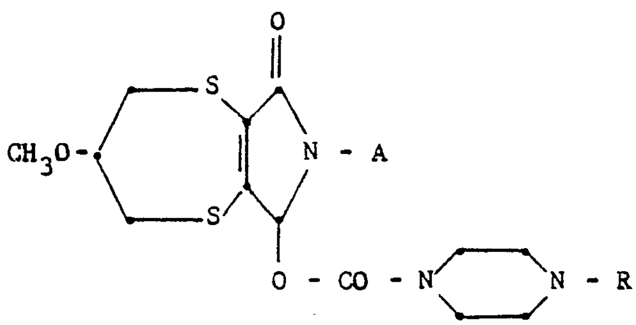

in which A represents a pyrid-2-yl, quinol-2-yl or 1,8-naphthyridin-2-yl radical, these radicals being optionally substituted by a halogen atom or an alkyl radical containing 1 to 4 carbon atoms or by an alkoxy radical of which the alkyl part contains 1 to 4 carbon atoms, and R represents a hydrogen atom or an alkyl radical containing 1 to 4 carbon atoms, or an alkanoyl radical containing 1 to 4 carbon atoms, its diastereoisomeric forms and their mixtures, in the form of a free base or of addition salts with acids, characterised in that a chlorocarbolylpiperazine of the general formula :

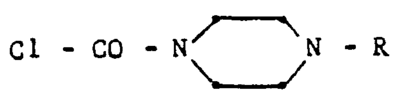

in which R is defined as in Claim 1, is reacted with a dithiepino [1,4] [2,3-c] pyrrole derivative of the general formula :

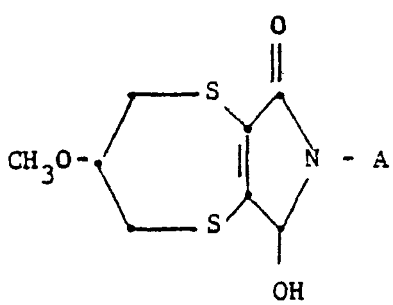

in which A is defined as herebefore or in that a piperazine of the general formula :

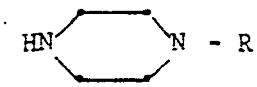

in which R is defined as in Claim 1 is reacted with a mixed carbonate of the general formula :

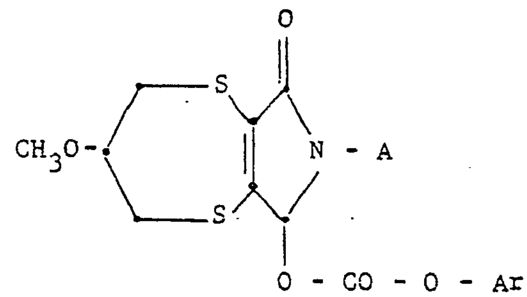

in which A is defined as herebefore and Ar represents a phenyl radical which is optionally substituted by an alkyl radical containing 1 to 4 carbon atoms or by a nitro radical, or where R represents an alkanoyl radical containing 1 to 4 carbon atoms, in that an acid or a reactive derivative of an acid of the general formula :

$$R_2—COOH$$

in which $R_2$ represents a hydrogen atom or an alkyl radical containing 1 to 3 carbon atoms is reacted with a product of the general formula :

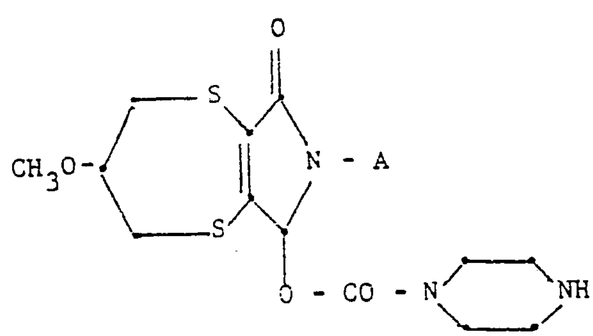

in which A is defined as herebefore or where R represents a hydrogen atom, in that a product of the general formula :

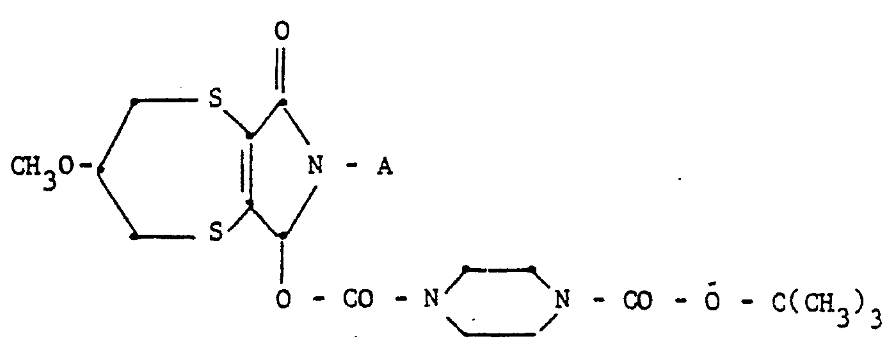

in which A is defined as herebefore is subjected to the action of trifluoroacetic acid, and thereafter, if desired, the product obtained is separated into its diastereoisomeric forms and, if desired, in converted to an addition salt with an acid.

**Ansprüche** (für die Vertragstaaten : BE, CH, DE, FR, GB, IT, LU, NL, SE)

1. Neues Derivat des Dithiepino [1,4] [2,3-c] pyrrols, dadurch gekennzeichnet, daß es der allgemeinen Formel

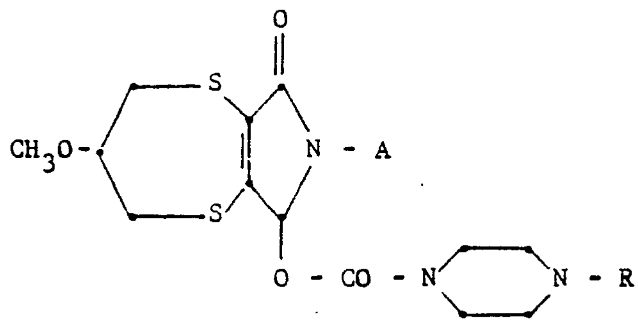

entspricht, worin A einen Rest Pyridyl-2, Chinolyl-2 oder 1,8-Naphtyridin-yl-2 bedeutet, wobei diese Reste gegebenenfalls substituiert sind durch ein Halogenatom oder durch einen Alkylrest mit 1 bis 4 Kohlenstoffatomen oder einen Alkyloxyrest, dessen Alkylteil 1 bis 4 Kohlenstoffatome enthält, und R bedeutet ein Wasserstoffatom oder einen Alkylrest mit 1 bis 4 Kohlenstoffatomen oder Alkanoyl mit 1 bis 4 Kohlenstoffatomen, seine diastereoisomeren Formen und deren Gemische, im Zustand der freien Base oder der Additionssalze mit Säuren.

2. Verfahren zur Herstellung eines Produkts gemäß Anspruch 1, dadurch gekennzeichnet, daß man ein Chlorcarbonylpiperazin der allgemeinen Formel

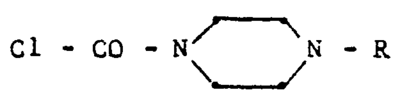

worin R wie in Anspruch 1 definiert ist, mit einem Derivat des Dithiepino [1,4] [2,3-c] pyrrols der allgemeinen Formel

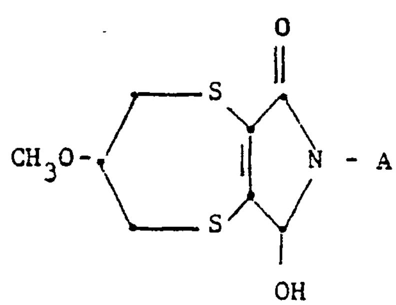

worin A wie in Anspruch 1 definiert ist, zur Reaktion bringt, und daß man gegebenenfalls das erhaltene Produkt in seine diastereoisomeren Formen auftrennt und es gegebenenfalls in ein Additionssalz mit einer Säure überführt.

3. Verfahren gemäß Anspruch 2, dadurch gekennzeichnet, daß man ein wie in Anspruch 2 definiertes Chlorcarbonylpiperazin mit einem Derivat des Dithiepino [1,4] [2,3-c] pyrrols in Form des Alkalisalzes, das gegebenenfalls in situ hergestellt ist, zur Reaktion bringt, wobei man in einem Organischen Lösungsmittel arbeitet, und daß man das erhaltene Produkt gegebenenfalls in seine diastereoisomeren Formen auftrennt und es gegebenenfalls in ein Additionssalz mit einer Säure überführt.

4. Verfahren gemäß Anspruch 2, dadurch gekennzeichnet, daß man ein wie in Anspruch 2 definiertes Chlorcarbonylpiperazin, gegebenenfalls in Salzform, mit einem Derivat des Dithiepino [1,4] [2,3-c] pyrrols, das wie in Anspruch 2 definiert ist, zur Reaktion bringt, wobei man in Gegenwart von pyridin und gegebenenfulls in Gegenwart einer tertiären organischen Base arbeitet, und daß man das erhaltene Produkt gegebenenfalls in seine diastereoisomeren Formen auftrennt und es gegebenenfalls in ein Additionssalz mit einer Säure überführt.

5. Verfahren zur Herstellung eines Produkts gemäß Anspruch 1, dadurch gekennzeichnet, daß man ein Piperazin der allgemeinen Formel

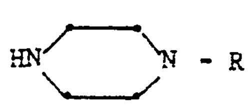

worin R wie in Anspruch 1 definiert ist, mit einem gemischten Carbonat der allgemeinen Formel

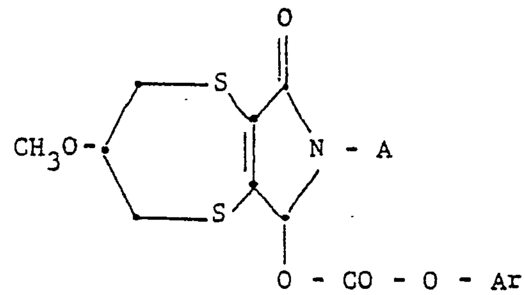

zur Reaktion bringt, worin A wie in Anspruch 1 definiert ist und Ar einen Phenylrest bedeutet, der gegebenenfalls durch einen Alkylrest mit 1 bis 4 Kohlenstoffatomen oder durch einen Nitrorest substituiert ist, und daß man das erhaltene Produkt gegebenenfalls in seine diastereoisomeren Formen auftrennt und es gegebenenfalls in ein Additionssalz mit einer Säure überführt.

6. Verfahren zur Herstellung eines Produkts gemäß Anspruch 1, worin R einen ALkanoylrest, mit 1 bis 4 Kohlenstoffatomen bedeutet, dadurch gekennzeichnet, daß man eine Säure oder ein reaktionsfähiges Derivat einer Säure der allgemeinen Formel

$$R_2\text{—COOH}$$

worin $R_2$ ein Wasserstoffatom oder einen Alkylrest mit 1 bis 3 Kohlenstoffatomen bedeutet, mit einem Produkt der allgemeinen Formel

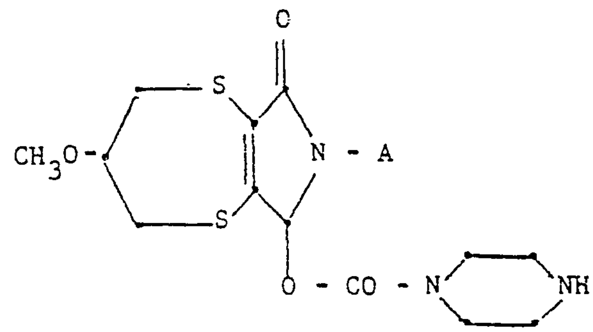

worin A wie in Anspruch 1 definiert ist, zur Reaktion bringt, und daß man das erhaltene Produkt gegebenenfalls in seine diastereoisomeren Formen auftrennt und es gegebenenfalls in ein Additionssalz mit einer Säure überführt.

7. Verfahren zur Herstellung eines Produktes gemäß Anspruch 1, worin R ein Wasserstoffatom bedeutet, dadurch gekennzeichnet, daß man ein Produkt der allgemeinen Formel

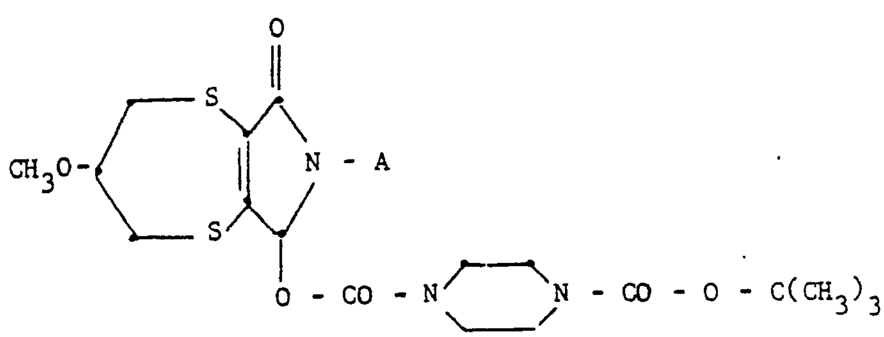

worin A wie in Anspruch 1 definiert ist, der Einwirkung von Trifluoressigsäure unterwirft, und daß man das erhaltene Produkt gegebenenfalls in seine diastereoisomeren Formen auftrennt, und es gegebenenfalls in ein Additionssalz mit einer Säure überführt.

8. Arzneimittel, dadurch gekennzeichnet, daß es ein Produkt gemäß Anspruch 1 in reinem Zustand oder zusammen mit einem oder mehreren Verdünnungs- oder Hilfsmitteln, die verträglich und pharmazeutisch annehmbar sind, enthält.

**Anspruch** (für den Vertragsstaat AT)

Verfahren zur Herstellung eines neuen Dithiepino [1,4] [2,3-c] pyrrol-derivats der allgemeinen Formel

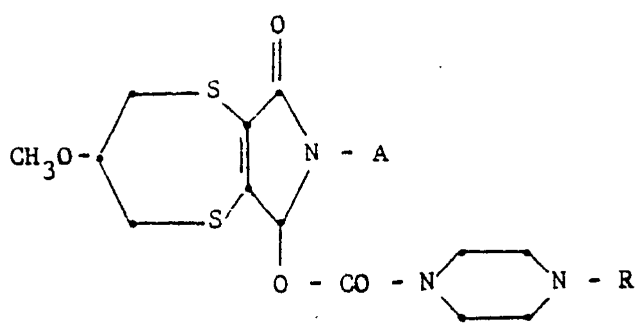

worin A einen 2-Pyridyl-, 2-Chinolyl- oder 1,8-Naphtyridin-2-yl-rest bedeutet, welche Reste gegebenenfalls durch ein Halogenatom oder durch eine Alkylgruppe mit 1 bis 4 Kohlenstoffatomen oder eine Alkyloxygruppe, die im Alkyteil 1 bis 4 Kohlenstoffatome enthält, substituiert sind, und R ein Wasserstoffatom oder einen Alkylrest mit 1 bis 4 Kohlenstoffatomen oder eine Alkanoylgruppe mit 1 bis 4 Kohlenstoffatomen darstellt, ihren diastereoisomeren Formen und deren Mischungen im Zustand der freien Base oder als Säureadditionssalze, dadurch gekennzeichnet, daß man ein Chlorcarbonylpiperazin der allgemeinen Formel

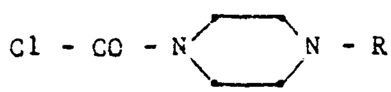

worin R die obige Bedeutung hat, mit einem Dithiepino [1,4] [2,3-c] pyrrol-derivat der allgemeinen Formel

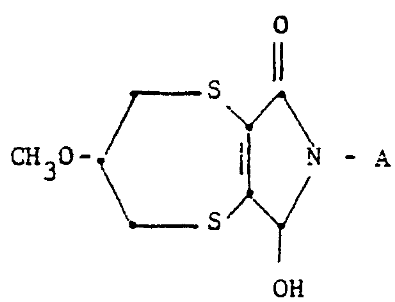

worin A die obige Bedeutung hat, reagieren läßt, oder daß man ein Piperazin der allgemeinen Formel

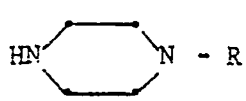

worin R die obige Bedeutung hat, mit einem gemischten Carbonat der allgemeinen Formel

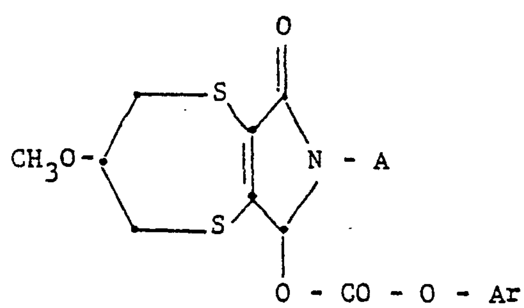

worin A die obige Bedeutung hat und Ar einen Phenylrest bedeutet, der gegebenenfalls durch eine Alkylgruppe mit 1 bis 4 Kohlenstoffatomen oder einen Nitrorest substituiert ist, reagieren läßt, oder daß man, wenn R eine Alkanoylgruppe mit 1 bis 4 Kohlenstoffatomen darstellt, eine Säure oder ein reaktionsfähiges Derivat einer Säure der allgemeinen Formel

$$R_2—COOH,$$

worin $R_2$ ein Wasserstoffatomen oder einen Alkylrest mit 1 bis 3 Kohlenstoffatomen darstellt, mit einer Verbindung der allgemeinen Formel

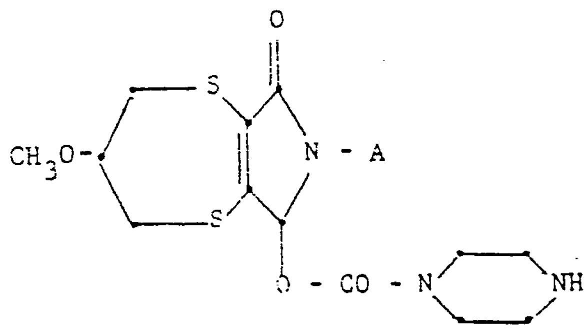

worin A die obige Bedeutung hat, reagieren läßt, oder daß man, wenn R ein Wasserstoffatom darstellt, eine Verbindung der allgemeinen Formel

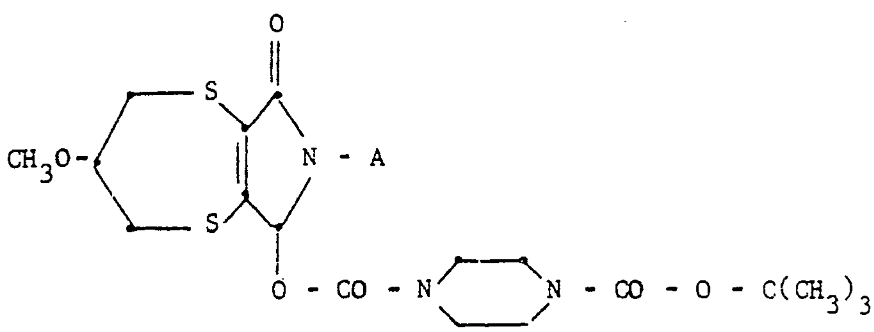

worin A die obige Bedeutung hat, mit Trifluoressigsäure behandelt, worauf man das erhaltene Produkt gegebenenfalls in seine diastereoisomeren Formen aufspaltet und gegebenenfalls in ein Säureadditionssalz überführt.